# EUROPEAN PATENT APPLICATION

(11) **EP 4 505 960 A1**
(43) Date of publication of application: **12.02.2025**
(21) Application number: 24192853.0
(22) Date of filing: 05.08.2024
(51) Int. Cl.: A61B 34/00

(54) **SURGICAL INSTRUMENT**

(30) Priority: 08.08.2023 JP 2023129598; 08.08.2023 JP 2023129608
(71) Applicant: Medicaroid Corporation, Kobe-shi, Hyogo 650-0047 (JP)
(72) Inventor: TAKAHASHI, Kaoru, Chuo-ku, Kobe-shi, 650-0047 (JP); KURIHARA, Gen, Minato-ku, Tokyo, 108-0075 (JP)
(74) Representative: Müller Hoffmann & Partner

(57) **Abstract**

A surgical instrument 40a includes: a first drive shaft 44a configured to roll a shaft 42, a second drive shaft 44b and a third drive shaft 44c configured to pitch a first joint 45a and yaw a second joint 45b, and a fourth drive shaft 44d configured to move a rod 47 in a longitudinal direction. The surgical instrument 40a further includes a lever 48 that includes an arm 481, an engagement portion 482 provided to one side of the arm and engaged with the rod 47, and a shaft portion 481a that is provided to the other side of the arm and is rotatably arranged on a base 411 so as to move the engagement portion by a driving force from the fourth drive shaft.

## Description

### BACKGROUND

The disclosure may relate to a surgical instrument.

In a related art, there has been a surgical instrument including drive shafts (for example, Patent Document 1: U.S. Patent No.7306597)

Patent Document 1 discloses a surgical instrument includes a drive shaft, a disk fixed to the drive shaft, an eccentric cam pin provided to the disk, a drive bar that is engaged with the eccentric cam pin, a pivot drive shaft that serves as a pivot axis of the drive bar, a core rod that moves back and forth in a longitudinal direction of an elongate shaft as the drive bar rotates about the pivot drive shaft, and blades of an end effector that opens and closes as the core rod moves back and forth. The surgical instrument disclosed in Patent Document 1 discloses four drive shafts extending vertically from a base, and among the four drive shafts, two drive shafts are used to move the core rod back and forth to drive the end effector, one drive shaft is used to rotate the elongate shaft.

Patent Document 1: U.S. Patent No.7,306,597

### SUMMARY

However, in Patent Document 1, the two drive shafts are used to move the core rod back and forth to drive the end effector, so there is an insufficient number of drive shafts to perform pitch and yaw joint movements of the end effector, which makes it difficult to perform pitch and yaw joint movements of the end effector. Accordingly, it may be difficult to roll the shaft, pitch and yaw the end effector, and move the rod (elongate element) to operate the end effector in the longitudinal direction of the shaft, by using the four drive shafts.

An object of an embodiment of the disclosure may be to provide a surgical instrument including four drive shafts that rolls a shaft, pitches and yaws an end effector, and moves an elongate element to move the end effector in a longitudinal direction of the shaft.

An aspect of the disclosure may be a surgical instrument that may include: a housing including a base that is to be attached to a robot arm; a shaft including a proximal end connected to the base; a wrist joint connected to a distal end side of the shaft, the wrist joint including a first joint configured to perform a pitch joint movement about a first rotation axis intersecting a longitudinal direction of the shaft and a second joint configured to perform a yaw joint movement about a second rotation axis intersecting the longitudinal direction and the first rotation axis; an end effector connected to a distal end of the wrist joint; an elongate element extending in the longitudinal direction of the shaft, the elongate element including a distal end thereof connected to the end effector; at least four drive shafts provided to the base and configured to driven to rotate by driving forces from motors provided to the robot arm; and a lever configured to move the elongate element in the longitudinal direction. The at least four drive shafts comprise a first drive shaft configured to roll the shaft about an axis along the longitudinal direction of the shaft, a second drive shaft and a third drive shaft configured to pitch the first joint and yaw the second joint, and a fourth drive shaft configured to move the elongate element in the longitudinal direction of the shaft. The lever includes an arm, an engagement portion provided to one side of the arm and engaged with the elongate element, and a shaft portion that is provided to the other side of the arm and is rotatably arranged on the base so as to move the engagement portion by the driving force from the fourth drive shaft.

According to the aspect, the shaft portion of the lever, which moves the elongate element in the longitudinal direction, serves as a rotation axis of the lever, and therefore there is no need to use one of the drive shafts as the rotation axis of the lever. Accordingly, only one of the drive shafts is used to move the elongate element in the longitudinal direction of the shaft to operate the end effector. As a result, it is possible to provide the surgical instrument including the four drive shafts that can roll the shaft, pitch and yaw the end effector, and move the elongate element to move the end effector in the longitudinal direction of the shaft.

According to the aspect, it is possible to roll the shaft, pitch and yaw the end effector, and move the elongate element in the longitudinal direction of the shaft to operate to the end effector, by the four drive shafts.

### BRIEF DESCRIPTION OF DRAWINGS

FIG. 1 is a diagram illustrating a configuration of a robotic surgical system according to an embodiment;
FIG. 2 is a block diagram illustrating a control-related configuration of the robotic surgical system according to an embodiment;
FIG. 3 is a diagram illustrating a perspective view of a state where a surgical instrument according to an embodiment is attached to a robot arm through an adaptor;
FIG. 4 is a diagram illustrating an exploded perspective view of a state where the surgical instrument according to of an embodiment is attached to the robot arm through the adaptor;
FIG. 5 is a diagram illustrating a perspective view of the surgical instrument according to an embodiment as seen from below.
FIG. 6 is a diagram illustrating a plan view of the surgical instrument according to an embodiment;
FIG. 7 is a diagram illustrating a perspective view of a state where a lid part of the surgical instrument according to an embodiment is detached;
FIG. 8 is a diagram illustrating a plan view of the state where the lid part of the surgical instrument according to an embodiment is detached;
FIG. 9 is a diagram illustrating a cross-sectional view of the state where the lid part of the surgical instrument according to an embodiment is detached;
FIG. 10 is a diagram illustrating a side view of an end effector, a wrist joint and a shaft according to an embodiment;
FIG. 11 is a diagram illustrating a cross-sectional view of the end effector, the wrist joint, and the shaft according to an embodiment;
FIG. 12 is a diagram illustrating a cross-sectional view of a third joint component taken along the arrow C1-C1 in FIG. 10;
FIG. 13 is a diagram illustrating an exploded perspective view of the end effector and a rod according to an embodiment;
FIG. 14 is a diagram illustrating a perspective view of a guide portion in the wrist joint of the surgical instrument according to an embodiment;
FIG. 15 is a diagram illustrating a perspective view of a memory board and a fixing portion according to an embodiment;
FIG. 16 is a diagram illustrating a cross-sectional view of the memory board and the fixing portion according to an embodiment;
FIG. 17 is a diagram illustrating a perspective view of the wrist joint of the surgical instrument according to an embodiment;
FIG. 18 is a diagram illustrating an exploded perspective view of the wrist joint of the surgical instrument according to an embodiment;
FIG. 19 is a diagram illustrating a cross-sectional view of first and fourth meshing portions of the wrist joint of the surgical instrument according to an embodiment;
FIG. 20 is a diagram illustrating a cross-sectional view illustrating second and third meshing portions of the wrist joint of the surgical instrument according to an embodiment;
FIG. 21 is a diagram illustrating an enlarged view of the first and fourth meshing portions of the wrist joint of the surgical instrument according to an embodiment;
FIG. 22 is a diagram illustrating an enlarged view of the second and third meshing portions of the wrist joint of the surgical instrument according to an embodiment;
FIG. 23 is a diagram illustrating a view of the wrist joint of the surgical instrument in an unrotated state according to an embodiment;
FIG. 24 is a diagram illustrating a view of the wrist joint of the surgical instrument in a state where the wrist joint is rotated by an angle θ1 according to an embodiment;
FIG. 25 is a diagram illustrating a view of the wrist joint of the surgical instrument in a state where the wrist joint is rotated by an angle θ2 according to an embodiment; and
FIG. 26 is a diagram illustrating a view of the wrist joint of the surgical instrument in a state where the wrist joint is rotated by an angle θ3 according to an embodiment.

### DETAILED DESCRIPTION

Descriptions are provided hereinbelow for embodiments based on the drawings. In the respective drawings referenced herein, the same constituents are designated by the same reference numerals and duplicate explanation concerning the same constituents is omitted. All of the drawings are provided to illustrate the respective examples only.

### (Configuration of Robotic Surgical System)

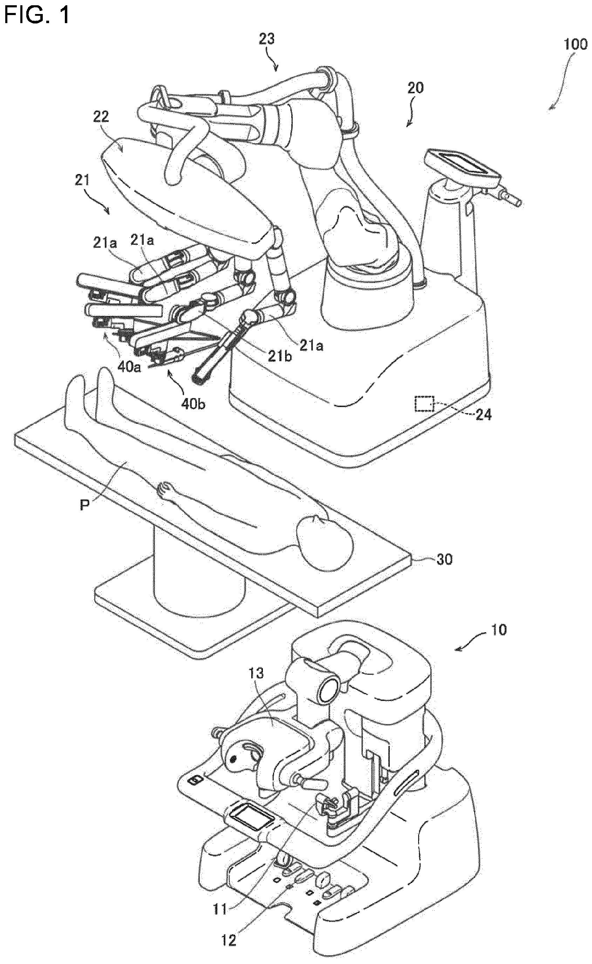

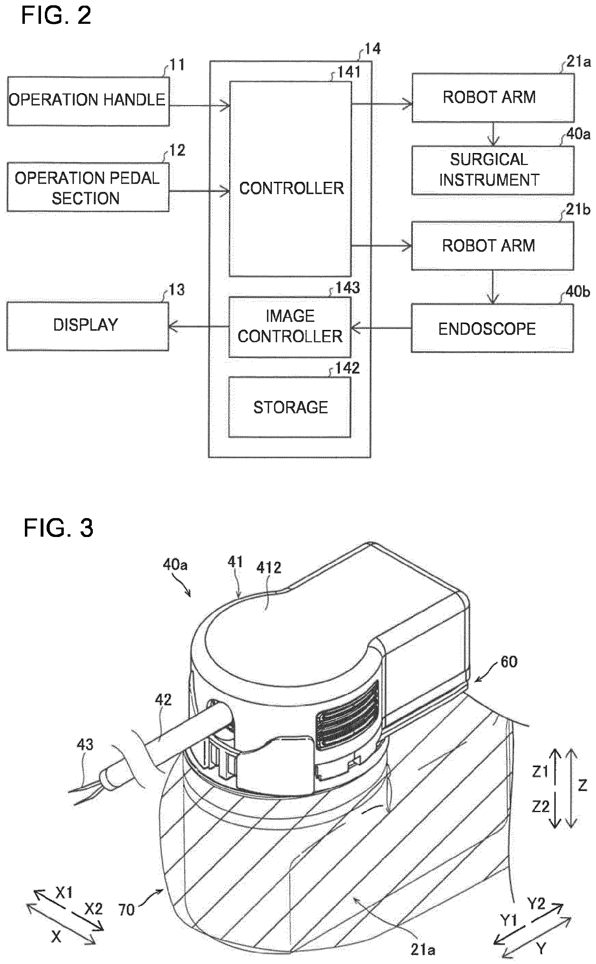

A configuration of a robotic surgical system 100 according to an embodiment is described with reference to FIGS. 1 and 2.

As illustrated in FIG. 1, the robotic surgical system 100 includes a remote control apparatus 10 and a patient-side apparatus 20. The remote control apparatus 10 is provided to remotely control medical equipment attached to the patient-side apparatus 20. When an operator, as a surgeon, inputs an action mode instruction to be executed by the patient-side apparatus 20, to the remote control apparatus 10, the remote control apparatus 10 transmits the action mode instruction to the patient-side apparatus 20 through a controller 24. In response to the action mode instruction transmitted from the remote control apparatus 10, the patient-side apparatus 20 operates the medical equipment, including surgical instruments 40a attached to robot arms 21a and an endoscope 40b attached to a robot arm 21b.

The patient-side apparatus 20 is positioned beside an operation table 30 on which a patient P is laid. The patient-side apparatus 20 includes plural robot arms 21a and 21b. One 21b of the robot arms holds the endoscope 40b and the other robot arms 21a hold the surgical instruments 40a. Each of the plural robot arms 21a and 21b includes plural joints. Each joint includes a driver (a driving device) including a servo-motor and a position detector such as an encoder or the like.

The arm base 22 is supported by a positioner 23 placed on the floor of an operation room. The positioner 23 includes a vertical articulated robot. The positioner 23 is configured to move the position of the arm base 22 three-dimensionally. The controller 24 is a control circuit including an arithmetic unit such as a CPU and/or the like, and a memory such as a ROM, a RAM, and/or the like.

The surgical instruments 40a as the medical equipment are detachably attached to distal ends of the robot arms 21a. The surgical instrument 40a includes a housing 41 (see FIG. 3), an elongate shaft 42 (see FIG. 3), and an end effector 43 (see FIG. 3). The end effector 43 may be grasping forceps, scissors, a hook, a highfrequency knife, a snare wire, a clamp, a stapler, a clip applier, an electric knife, or a needle, for example. The end effector 43 is not limited to those and can be various types of treatment tools. In surgeries using the patient-side apparatus 20, the robot arms 21a introduce the surgical instruments 40a into the body of the patient P through a cannula (trocar) placed on the body surface of the patient P.

To the distal end of the robot arm 21b, the endoscope 40b as the medical equipment is detachably attached. The endoscope 40b captures an image in a body cavity of the patient P. The captured image is outputted to the remote control apparatus 10. The endoscope 40b may be a 3D endoscope capable of capturing a three-dimensional image or a 2D endoscope. In surgeries using the patient-side apparatus 20, the robot arm 21b introduces the endoscope 40b into the body of the patient P through a trocar placed on the body surface of the patient P.

The remote control apparatus 10 is an apparatus that allows the operator to operate the medical equipment attached to the robot arms 21a and 21b. Specifically, the remote control apparatus 10 is configured to transmit action mode instructions which are inputted by the operator and are to be executed by the surgical instruments 40a and the endoscope 40b, to the patient-side apparatus 20 through the controller 24.

The action modes to be executed by the surgical instruments 40a include modes of actions to be taken by each surgical instrument 40a (a series of positions and postures) and actions to be executed by the function of each surgical instrument 40a. For example, in a case in which the surgical instrument 40a is a vessel sealer, the operational modes to be performed by the surgical instrument 40a include pitch and yaw rotations of the wrist of the end effector 43, opening and closing of the jaws, coagulating tissue by supplying a coagulation current to the end effector, and incising tissue by supplying a cutting current to the end effector.

The action modes to be executed by the endoscope 40b include the position and posture of the distal end of the endoscope 40b and setting of the zoom magnification, for example.

As illustrated in FIGS. 1 and 2, the remote control apparatus 10 includes operation handles 11, an operation pedal section 12, a display 13, and a control apparatus 14.

The operation handles 11 are provided in order to remotely operate medical equipment attached to the robot arms 21a. Specifically, the operation handles 11 accept operations by the operator for operating the medical equipment (the surgical instruments 40a and endoscope 40b). The operation handles 11 are composed of two operation handles 11 arranged side by side in the horizontal direction.

The operation handles 11 extend from the rear side of the remote control apparatus 10 toward the front side. The operation handles 11 are configured to move in a predetermined three-dimensional operation region.

The remote control apparatus 10 and the patient-side apparatus 20 constitute a master-slave system in terms of controlling movements of the robot arms 21a and 21b. The operation handles 11 constitute a master side operating part in the master-slave system, and the robot arms 21a and 21b to which the medical equipment is attached constitute a slave side operating part in the master-slave system. When the operator operates the operation handles 11, the movement of one of the robot arms 21a or 21b is controlled so that the distal end portion (the end effector 43 of the surgical instrument 40a) of the robot arm 21a or the distal end portion (the endoscope 40b) of the robot arm 21b moves following the movement of the operation handles 11.

The operation pedal section 12 or an operation pedal unit includes plural pedals to execute medical equipment-related functions. The plural pedals include a coagulation pedal, a cutting pedal, a camera pedal, and a clutch pedal. The plural pedals are operated by a foot of the operator.

By operating the coagulation pedal, a coagulation current is supplied to the surgical instrument 40a so as to coagulate the surgical site. By operating the cutting pedal, a cutting current is supplied to the surgical instrument 40a so as to cut the surgery site.

By operating the camera pedal, the operation handles 11 are allowed to operate the endoscope 40b. That is, the position and orientation of the endoscope 40b are controllable by the operation handles 11 while the camera pedal is being pressed. The endoscope 40b is controlled by using both of the right and left operation handles 11, for example.

The clutch pedal is used to temporarily disconnect operation-related connection between the operation handles 11 and the robot arms 21a to stop movements of the surgical instruments 40a. Specifically, when the clutch pedal is being pressed, the robot arms 21a of the patient-side apparatus 20 do not work even if the operation handles 11 are operated.

The display 13 (or a display device) is configured to display images captured by the endoscope 40b. The display 13 comprises a scope type display or a non-scope type display. (Note that FIG. 1 illustrates a scope type display 13.) The scope type display is a display configured in such a manner that the operator looks into the display. The non-scope type display is a display like an open-type display that includes a flat screen and the operator is able to see without looking into, such as normal displays for personal computers.

The display 13 displays a 3D image captured by the endoscope 40b attached to the robot arm 21b of the patient-side apparatus 20. Note that the display 13 may display a 2D image captured by the endoscope 40b provided to the patient-side apparatus 20.

As illustrated in FIG. 2, the control apparatus 14 includes a controller 141, a storage 142, and an image controller 143, for example. The controller 141 includes an arithmetic unit such as a CPU. The storage 142 includes a memory, such as a ROM and a RAM. The control apparatus 14 may be composed of a single control apparatus performing centralized control or may be composed of plural control apparatuses that perform decentralized control in cooperation with each other. The controller 141 determines whether an action mode instruction inputted by the operation handles 11 is to be executed by the robot arms 21a or to be executed by the endoscope 40b, depending on the state of the operation pedal section 12. When determining that the action mode instruction inputted by the operation handles 11 is to be executed by any one of the surgical instruments 40a, the controller 141 transmits the action mode instruction to the corresponding robot arm 21a through the controller 24. The robot arm 21a is thereby driven by the controller 24 and thus the movement of the surgical instrument 40a attached to the robot arm 21a is controlled.

The storage 142 stores control programs corresponding to the types of the surgical instrument 40a, for example. The controller 141 reads the stored control programs according to the types of the attached surgical instruments 40a. The action mode instructions from the operation handles 11 and/or the operation pedal section 12 of the remote control apparatus 10 thereby cause the respective surgical instruments 40a to perform proper movements.

The image controller 143 transmits images acquired by the endoscope 40b to the display 13. The image controller 143 performs processing and modifying the images when needed.

### (Configurations of Surgical Instrument, Adaptor, Drape, and Robot Arm)

With reference to FIGS. 3 to 5, configurations of the surgical instrument 40a, an adaptor 60, a drape 70, and the robot arm 21a are described.

Here, a longitudinal direction of the surgical instrument 40a (a longitudinal direction of the shaft 42) is defined as the Y direction, the distal side (the side toward the end effector 43) of the surgical instrument 40a along the Y direction is defined as the Y1 direction, and the opposite side of the Y1 direction is defined as the Y2 direction. The direction in which the surgical instrument 40a and the adaptor 60 are adjacent to each other is defined as a Z direction, the surgical instrument 40a side along the Z direction is defined as a Z1 direction, and the opposite side of the Z1 direction is defined as a Z2 direction. Further, the direction orthogonal to the Y direction and the Z direction is referred to as an X direction, one side along the X direction is referred as an X1 direction, and the other side along the X direction is referred to as an X2 direction.

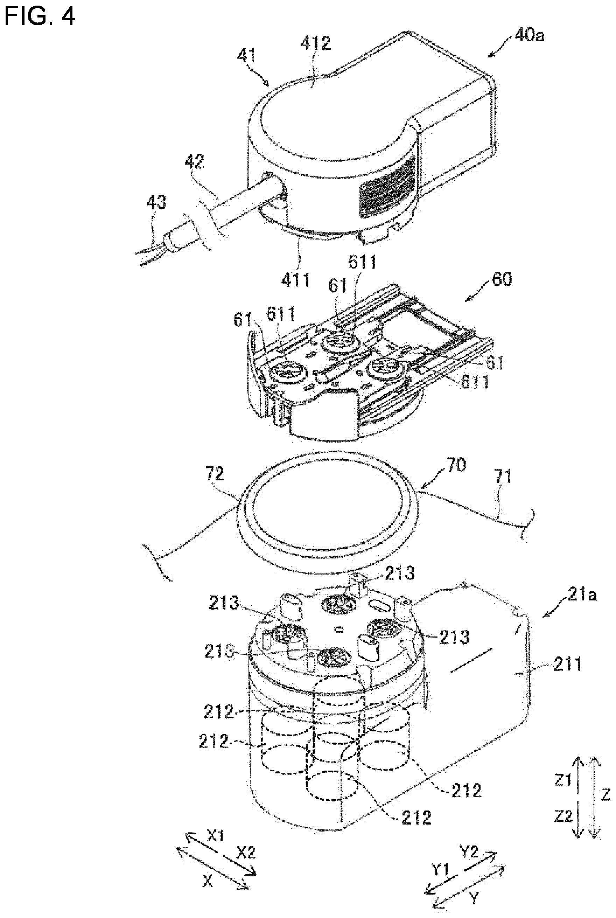

As illustrated in FIGS. 3 and 4, the surgical instrument 40a is detachably attached to the robot arm 21a of the robotic surgical system 100. Specifically, the surgical instrument 40a is detachably attached to the robot arm 21a via the adaptor 60. The adaptor 60 is a drape adaptor configured to sandwich a sterile drape 70 to cover the robot arm 21a, in conjunction with the robot arm 21a.

The surgical instrument 40a is attached to the Z1 side of the adaptor 60. The adaptor 60 is attached to the Z1 side of the robot arm 21a.

As illustrated in FIG. 4, the drape 70 includes a body section 71 that covers the robot arm 21a and an attachment section 72 sandwiched between the robot arm 21a and the adaptor 60. The body section 71 is made of a flexible film member. The body section 71 includes an opening so that the robot arm 21a is engaged with the adaptor 60. To the opening of the body section 71, the attachment section 72 is provided. The attachment section 72 is made of a resin mold member. The attachment section 72 is harder (less flexible) than the body section 71. The attachment section 72 includes an opening so that the robot arm 21a is engaged with the adaptor 60.

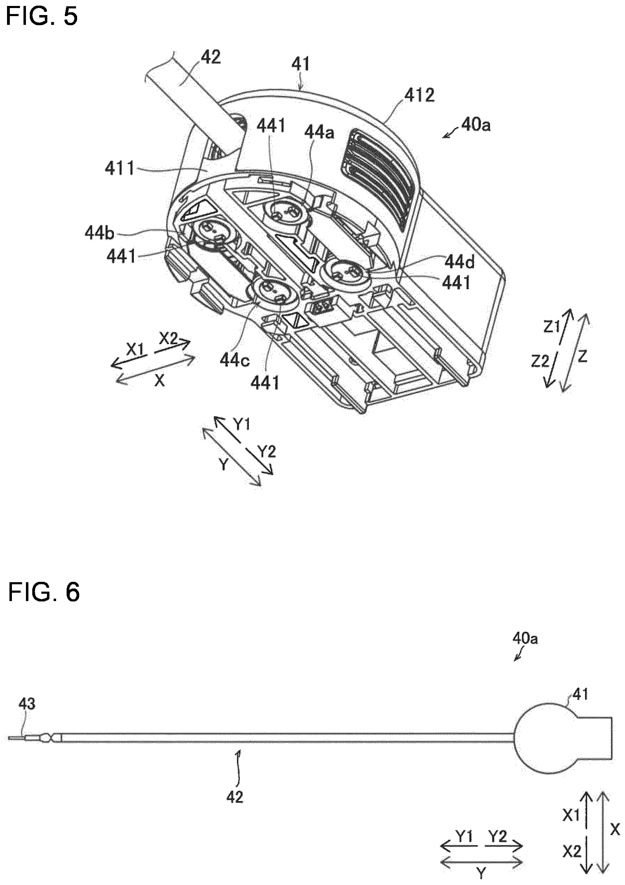

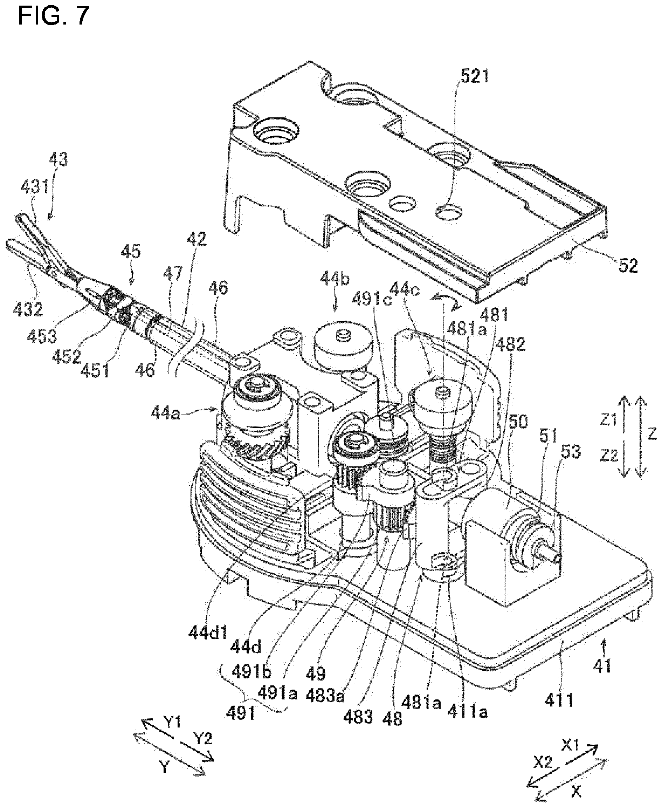

As illustrated in FIGS. 5 and 7, the surgical instrument 40a includes a plurality (four) of drive shafts 44a, 44b, 44c and 44d. The drive shafts 44a, 44b, 44c, and 44d are provided within the housing 41 and are rotatable about respective rotation axes thereof extending along the Z axis. The drive shafts 44a to 44d are rotationally driven by driving forces from motors of drivers 212 (driving devices 212) provided in the robot arm 21a. The drive shafts 44a to 44d are provided for operating (driving) the elongate shaft 42, the end effector 43, and a wrist joint 45 (described later).

The drive shafts 44a to 44d respectively include protrusions 441 engaged with corresponding drive transmission members 61 (described later) of the adaptor 60 so as to transmit the driving forces from the robot arm 21a to the shaft 42, the end effector 43, and the wrist joint 45. The protrusions 441 protrude from the Z2 side surfaces of the drive shafts 44a to 44d toward the adaptor 60 (the Z2 side). Each of the protrusion 441 includes plural protrusion portions that arranged in a straight line.

As illustrated in FIG. 4, the adaptor 60 includes the drive transmission members 61. The drive transmission members 61 are configured to transmit the driving forces from the robot arm 21a to the drive shafts 44a to 44d of the surgical instrument 40a. In other words, the drive transmission members 61 respectively provided to correspond to the drive shafts 44a to 44d of the surgical instrument 40a. The drive transmission members 61 are rotatable about respective rotation axes thereof, which extend along the Z direction.

The drive transmission members 61 include engagement recesses 611 that are respectively engaged with the protrusions 441 of the drive shafts 44a to 44d of the surgical instrument 40a. The engagement recess 611 is located on the surgical instrument 40a side (the Z1 side) of the drive transmission member 61 and is recessed from the Z1 side surface of the drive transmission member 61 toward the Z2 side, opposite to the surgical instrument 40a side. Each of the drive transmission members 61 further includes an engagement recess provided on the Z2 side surface thereof and is configured to be engaged with a corresponding engagement protrusion 213 (described later) of the robot arm 21a.

The robot arm 21a includes a frame 211, the drivers 212, and the engagement protrusions 213. The plural (four) drivers 212 are provided to correspond to the plural drive shafts 44a to 44d of the surgical instrument 40a and correspond to the plural drive transmission members 61 of the adaptor 60. Each of the drivers 212 includes an absolute encoder and a servomotor and is configured to drive the corresponding engagement protrusion 213 to rotate about rotational axes thereof extending in the Z direction. The engagement protrusion 213 of the driver 212 is engaged with the engagement recess provided on the Z2 side surface of the corresponding drive transmission member 61. The drivers 212 are configured to drive the drive transmission members 61 of the adaptor 60, which are respectively engaged with the engagement protrusions 213 of the drivers 212, to rotate about the rotational axes of the drive transmission members 61 extending in the Z direction, so as to drive the drive shafts 44a to 44d of the surgical instrument 40a, which are respectively engaged with the drive transmission members 61, to rotate about the rotational axes of the drive shafts 44a to 44d extending in the Z direction.

### (Detailed Configuration of Surgical Instrument)

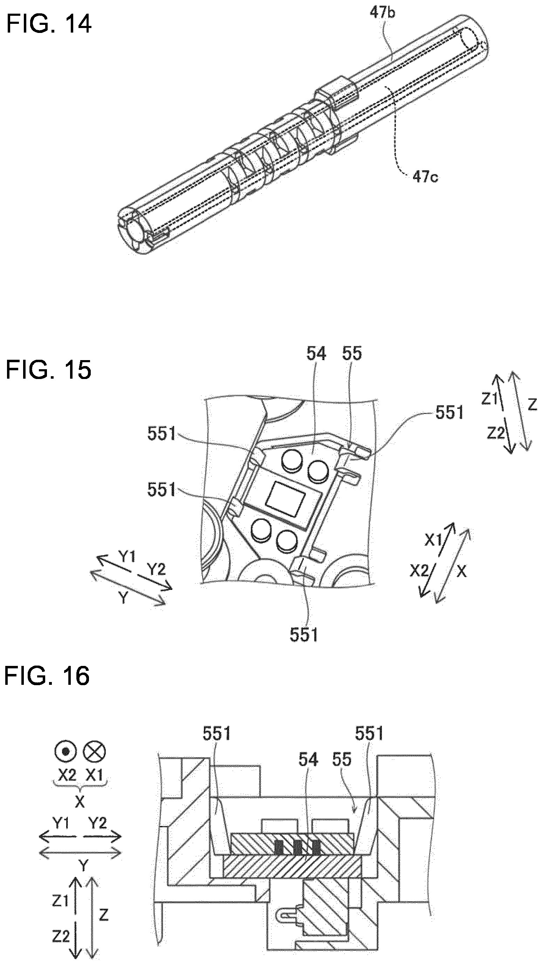

With reference to FIGS. 6 to 26, the configuration of the surgical instrument 40a is described in detail below.

As illustrated in FIGS. 6 to 26, the surgical instrument 40a includes the housing 41, the elongate shaft 42, the end effector 43, and the plural drive shafts 44a to 44d. The surgical instrument 40a also includes the wrist joint 45, wires 46, a rod 47, a lever 48, a driving force transmission part 49, a holding member 50 (a retainer 50), and a spring member 51. Note that the rod 47 is an example of an elongate element.

As illustrated in FIG. 7, the housing 41 includes a base 411 and a lid part 412 (see FIG. 4). The base 411 is attached to the robot arm 21a (see FIG. 4) via the adaptor 60. The base 411 is provided with the plural drive shafts 44a to 44d. The lid part 412 removably covers the base 411. Specifically, the lid part 412 covers the base 411 from the side (the Z1 side) opposite to a mounting surface of the base 411 to the robot arm 21a.

The shaft 42 is provided to extend in the Y direction. A proximal end (an end on the Y2 side) of the shaft 42 is connected to the base 411. A distal end (an end on the Y1 side) of the shaft 42 is connected to a proximal end of the wrist joint 45.

The end effector 43 is connected to a distal end of the wrist joint 45. The end effector 43 includes a first jaw 431 and a second jaw 432.

As illustrated in FIGS. 7 to 9, the drive shafts 44a to 44d include a first drive shaft 44a for rolling the elongate shaft 42 about an axis thereof extending in the Y direction, a second drive shaft 44b and a third drive shaft 44c for pitching a first joint 45a about a rotation axis A1 and yawing a second joint 45b about a rotation axis A2, and a fourth drive shaft 44d for moving the rod 47 in the Y direction.

The first drive shaft 44a is connected to a gear 422 that is connected to the proximal end of the shaft 42. When the first drive shaft 44a is driven to rotate, the elongate shaft 42 is driven to rotate in a rolling manner by the first drive shaft 44a via the gear 422. The second drive shaft 44b and the third drive shaft 44c are connected to the wires 46 that are connected to the wrist joint 45. When the second drive shaft 44b and the third drive shaft 44c are driven to rotate, the wrist joint 45 is caused to perform pitch or yaw joint motion via the wires 46. Note that the movement of the rod 47 in the Y direction by the fourth drive shaft 44d will be described later.

As illustrated in FIGS. 7, 10 and 11, the wrist joint 45 is connected to the distal end side (Y1 side) of the shaft 42 and is configured to articulate with respect to the shaft 42. Specifically, the wrist joint 45 includes a first joint component 451, a second joint component 452, and a third joint component 453. The first joint component 451 is connected to the distal end side (Y1 side) of the shaft 42. The second joint component 452 is connected to the first joint component 451 via the first joint 45a. The second joint component 452 is disposed on the distal end side (Y1 side) of the first joint component 451, and is meshed with the first joint component 451 so as to perform a pitch joint movement. The third joint component 453 is connected to the second joint component 452 via the second joint 45b. The third joint component 453 is disposed on the distal end side of the second joint component 452 and is meshed with the second joint component 452 so as to perform a yaw joint movement. The third joint component 453 has a shape whose diameter gradually decreases toward the distal end side (Y1 side) in order to connect the shaft 42 and the end effector 43 which has a smaller diameter than that of the shaft 42. The wrist joint 45 will be described in detail later. Note that the first joint component 451, the second joint component 452, and the third joint component 453 are examples of a first joint component, a second joint component, and a third joint component, respectively.

The wires 46 are provided to drive the wrist joint 45. The wires 46 allow the wrist joint 45 to perform the pitch and yaw joint movements. The wires 46 are provided inside the shaft 42 and extend in the Y direction and the number of the wires 46 provided is four. Two of the four wires 46 connect the wrist joint 45 and the second drive shaft 44b to each other, and the other two of the four wires 46 connect the wrist joint 45 and the third drive shaft 44c to each other.

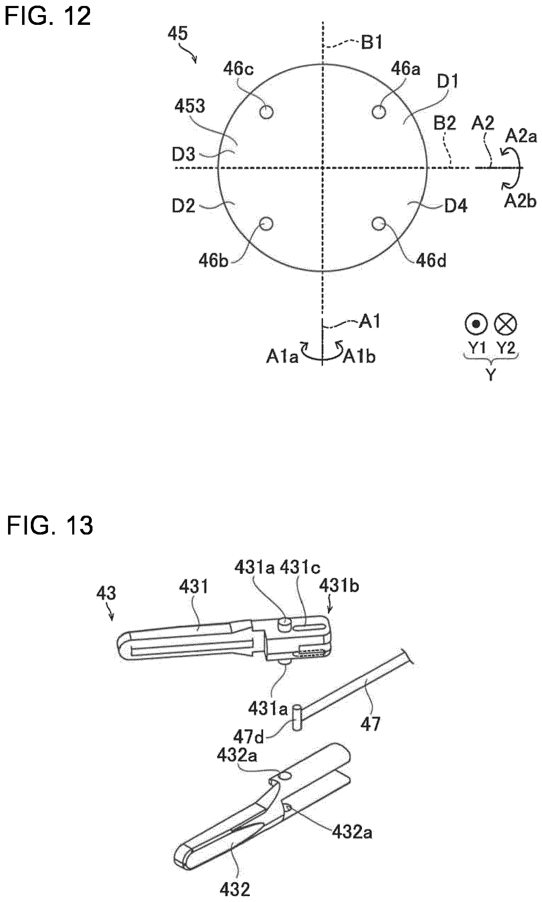

For example, as illustrated in FIG. 12, the four wires 46 include wires 46a, 46b, 46c, and 46d. The wires 46a, 46b, 46c and 46d have distal ends thereof fixed to the third joint component 453. Proximal ends of the wires 46a and 46b are wound around and fixed to the second drive shaft 44b. The wires 46a and 46b are wound around the second drive shaft 44b in opposite directions. In other words, when the second drive shaft 44b rotates in one direction, one of the wires 46a and 46b is pulled in the proximal direction (Y2 direction), and the other of the wires 46a and 46b is released and pulled out in the distal direction (Y1 direction). To the contrary, when the second drive shaft 44b rotates in the other direction opposite to the one direction, the other of the wires 46a and 46b is pulled in the proximal direction (Y2 direction), and the one of the wires 46a and 46b is released and pulled out in the distal direction (Y1 direction). Note that the wires 46a, 46b, 46c, and 46d are examples of a first wire, a second wire, a third wire, and a fourth wire, respectively.

Proximal ends of the wires 46c and 46d are wound around and fixed to the third drive shaft 44c. The wires 46c and 46d are wound around the third drive shaft 44c in opposite directions. In other words, when the third drive shaft 44c rotates in one direction, one of the wires 46c and 46d is pulled in the proximal direction (Y2 direction), and the other of the wires 46c and 46d is released and pulled out in the distal direction (Y1 direction). To the contrary, when the third drive shaft 44c rotates in the other direction opposite to the one direction, the other of the wires 46c and 46d is pulled in the proximal direction (Y2 direction), and the one of the wires 46c and 46d is released and pulled out in the distal direction (Y1 direction).

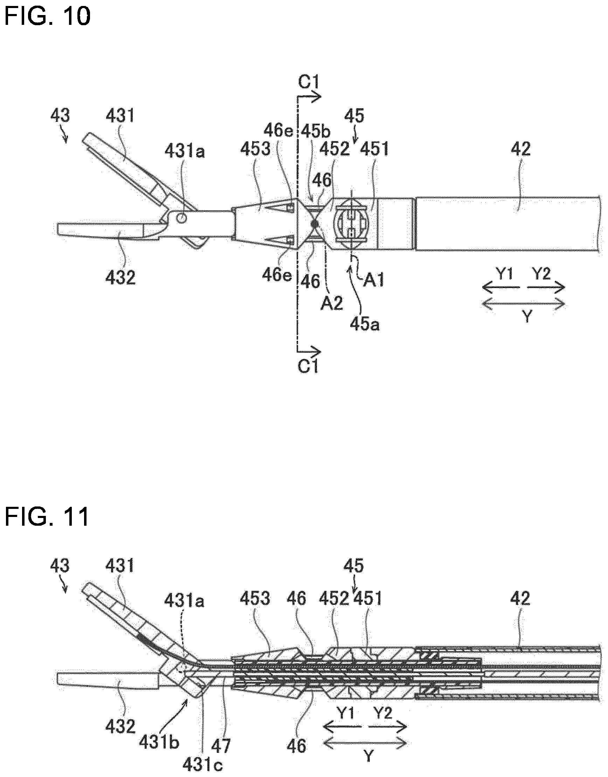

As illustrated in FIG. 10, the wrist joint 45 includes the first joint 45a that rotates (pitches) about the rotation axis A1 orthogonal to the longitudinal direction of the shaft 42 so as to perform the pitch joint movement, and the second joint 45b that rotates (yaws) about the rotation axis A2 orthogonal to the longitudinal direction of the shaft 42 and the rotation axis A1 so as to perform the yaw joint movement. The wires 46 cause the wrist joint 45 to pitch and yaw, by driving the wires 46a and 46b by the second drive shaft 44b and by driving the wires 46c and 46d by the third drive shaft 44c. The wrist joint 45 is driven by the second drive shaft 44b and the third drive shaft 44c to perform the rotation (the pitch joint movement) about the rotation axis A1 of the first joint 45a and the rotation (the yaw joint movement) about the rotation axis A2 of the second joint 45b. The second drive shaft 44b is configured to rotate in a first direction to apply tension to the wire 46a and to rotate in a second direction opposite the first direction to apply tension to the wire 46b. The third drive shaft 44c is configured to rotate in a third direction to apply tension to the wire 46c, and to rotate in a fourth direction opposite to the third direction to apply tension to the wire 46d. When the rotation of the second drive shaft 44b applies the tension to the wire 46a and the rotation of the third drive shaft 44c applies the tension to the wire 46d, or when the rotation of the second drive shaft 44b applies the tension to the wire 46b and the rotation of the third drive shaft 44c applies the tension to the wire 46c, the first joint 45a performs the pitch joint movement about the rotation axis A1. When the rotation of the second drive shaft 44b applies the tension to the wire 46a and the rotation of the third drive shaft 44c applies the tension the wire 46c, or when the rotation of the second drive shaft 44b applies the tension to the wire 46b and the rotation of the third drive shaft 44c applies the tension to the wire 46d, the second joint 45b performs the yaw joint movement about the rotation axis A2. Note that the rotation axis A1 and the rotation axis A2 are examples of a rotation axis A1 and a rotation axis A2, respectively.

Specifically, as illustrated in FIG. 12, when the wire 46a is pulled and the wire 46b is loosened by the second drive shaft 44b and the wire 46d is pulled and the wire 46c is loosened by the third drive shaft 44c, the first joint 45a rotates in the A1b direction about the rotation axis A1. Further, when the wire 46b is pulled and the wire 46a is loosened by the second drive shaft 44b and the wire 46c is pulled and the wire 46d is loosened by the third drive shaft 44c, the first joint 45a rotates in the A1a direction about the rotation axis A1.

To the contrary, when the wire 46a is pulled and the wire 46b is loosened by the second drive shaft 44b and the wire 46c is pulled and the wire 46d is loosened by the third drive shaft 44c, the second joint 45b rotates in the A2a direction about the rotation axis A2. Further, when the wire 46b is pulled and the wire 46a is loosened by the second drive shaft 44b and the wire 46d is pulled and the wire 46c is loosened by the third drive shaft 44c, the second joint 45b rotates in the A2b direction about the rotation axis A2.

The four wires 46a to 46d have respective ends 46e (see FIG. 10) fixed to the third joint component 453 at the vicinity of the proximal end of the third joint component 453. FIG. 12 is a cross-sectional view of the third joint component 453 taken along the line C1-C1 of FIG. 10. As illustrated in FIG. 12, a cross section of the third joint component 453 includes four regions, which are a first region D1 to a fourth region D4, divided by a plane B1 parallel to both the longitudinal direction (Y direction) of the shaft 42 and the rotation axis A1, and a plane B2 parallel to both the longitudinal direction (Y direction) of the shaft 42 and the rotation axis A2. The four wires 46a to 46d pass through the first region D1 to the fourth region D4, respectively, and have the ends 46e thereof fixed to the vicinity of the proximal end of the third joint component 453. Specifically, the wire 46a passes through the first region D1 at a position offset from planes B1 and B2, the wire 46b passes through the second region D2 at a position offset from planes B1 and B2, the wire 46c passes through the third region D3 at a position offset from planes B1 and B2, and the wire 46d passes through the fourth region D4 at a position offset from planes B1 and B2. As illustrated in FIG. 12, the first region D1 is arranged in a first quadrant, the second region D2 is arranged in a third quadrant, the third region D3 is arranged in a second quadrant, and the fourth region D4 is arranged in a fourth quadrant. Note that the plane B1 and the plane B2 are examples of a first plane and a second plane, respectively.

As illustrated in FIGS. 7 to 9 and 11, the rod 47 is provided for driving the end effector 43. The rod 47 is provided inside the shaft 42 and extends along the Y direction in the shaft 42. A distal end of the rod 47 is connected to the end effector 43. Specifically, the distal end of the rod 47 is connected to the first jaw 431.

As illustrated in FIGS. 11 and 13, the first jaw 431 includes a support shaft 431a that is rotatably supported by the second jaw 432 and a connection portion 431b that is connected to the distal end of the rod 47, such that the first jaw 431 rotates about an axis of the support shaft 431a due to the movement of the rod 47 in the Y direction. With this configuration, the first jaw 431 is rotated about the axis of the support shaft 431a by moving the rod 47 in the Y direction, so that the first jaw 431 is opened or closed relative to the second jaw 432. The end effector 43 is of a single-opening type in which the first jaw 431 opens and closes relative to the second jaw 432.

The support shaft 431a includes a pair of shaft portions that are provided on both sides of the first jaw 431. The pair of shaft portions of the support shaft 431a are inserted in and rotatably supported by a pair of holes 432a provided to the second jaw 432. The connection portion 431b includes a long hole portion 431c. The long hole portion 431c includes a pair of long holes 431c. A pin portion 47d is provided at the distal end of the rod 47 and extends in a direction orthogonal to the Y direction. One end and the other end of the pin portion 47d of the rod 47 are inserted in and supported by the pair of long holes 431c of the connection portion 431b of the first jaw 431 so that the pin portion 47d of the rod 47 is slidable along the long holes 431c of the connection portion 431b of the first jaw 431. When the rod 47 is moved in the Y1 direction, the pin portion 47d of the rod 47 slides along the long holes 431c, so that a force is applied in a direction in which the first jaw 431 opens. As a result, the first jaw 431 rotates about the axis of the support shaft 431a in the opening direction of the first jaw 431. To the contrary, when the rod 47 is moved in the Y2 direction, the pin portion 47d of the rod 47 slides along the long holes 431c, so that a force is applied in a direction in which the first jaw 431 closes. As a result, the first jaw 431 rotates about the axis of the support shaft 431a in the closing direction.

At the wrist joint 45, the rod 47 needs to bend as the wrist joint 45 articulates. Accordingly, the rod 47 is guided by a guide portion 47b provided to the wrist joint 45 as illustrated in FIG. 13. Specifically, a part of the rod 47 that passes through the wrist joint 45 is made of a wire 47c and is configured to be bendable. The guide portion 47b is made of resin and is configured to be bendable. The guide portion 47b guides the movement of the wire 47c along the Y direction so as to prevent the wire 47c from buckling. This allows the force generated by the movement of the rod 47 in the Y direction to be transmitted with preventing the force from losing.

In an embodiment, as illustrated in FIGS. 7 to 9, the lever 48 moves the rod 47 in the Y direction. The lever 48 includes an arm 481, an engagement portion 482 provided to one side of the arm 481 and engaged with the rod 47, and a shaft portion 481a that is provided to the other side of the arm 481 and is rotatably arranged on the base 411 so as to move the engagement portion 482 by the driving force from the fourth drive shaft 44d. In other words, the lever 48 includes a shaft portion 481a that rotates about an axis thereof by the driving force from the fourth drive shaft 44d, an arm 481 provided to the shaft portion 481a, and an engagement portion 482 provided to the arm 481 and engaged with the rod 47. With this configuration, the shaft portion 481a of the lever 48, which moves the rod 47 in the Y direction, serves as the rotation axis of the lever 48, so there is no need to use one of the drive shafts as the rotation axis of the lever. As a result, the rod 47 can be moved in the Y direction of the shaft 42 using only one drive shaft (e.g., the fourth drive shaft 44d), to operate the end effector 43. As a result, the four drive shafts 44a to 44d can rotate the elongate shaft 42 in roll, articulate the end effector 43 in pitch and yaw, and also move the rod 47 in the Y direction of the shaft 42 for operating the end effector 43.

In an embodiment, the arm 481 of the lever 48 includes a pair of arms 481 opposed to each other in the Z direction. The pair of arms 481 include a Z1 side arm 481, and a Z2 side arm 481 that is opposes to the Z1 side arm 481 in the Z direction. The engagement portion 482 of the lever 48 includes a Z1 side engagement portion 482 provided to the Z1 side arm 481 and a Z2 side engagement portion 482 provided to the Z2 side arm 481. The Z1 side engagement portion 482 is engaged with the rod 47 from one side (the Z1 side). The Z2 side engagement portion 482 is engaged with the rod 47 from the other side (the Z2 side). This allows the engagement portions 482 to be engaged with the rod 47 from the one side and the other side of the rod 47, so that the driving force of the fourth drive shaft 44d can be reliably transmitted to the rod 47 via the lever 48. Note that the Z1 side arm 481 and the Z2 side arm 481 are examples of a first arm and a second arm, respectively. The Z1 side engagement portion 482 and the Z2 side engagement portion 482 are examples of a first engagement portion and a second engagement portion, respectively.

In an embodiment, the Z1 side engagement portion 482 is engaged with the rod 47 from the one side (the Z1 side) via the holding member 50. The Z2 side engagement portion 482 is engaged with the rod 47 from the other side (the Z2 side) via the holding member 50. With this configuration, the engagement portions 482 are engaged with the rod 47 from the one side and the other side of the rod 47 via the holding member 50, so there is no need to provide, to the rod 47, an engagement portion(s) to be engaged with the engagement portions 482. As a result, the structure of the rod 47 can be simplified.

The pair of arms 481 are spaced apart from each other in the Z direction and connected to each other by a connection portion 483 extending in the Z direction. Each of the pair of arms 481 is provided to extend in the X direction from the connection portion 483 to the position of the rod 47. Each of the pair of arms 481 includes a shaft portion 481a between an end thereof on the connection portion 483 side and an end thereof on the rod 47 side. Specifically, each arm 481 includes the shaft portion 481a at a position closer to the connection portion 483 from the center between the connection portion 483 side end portion and the rod 47 side end portion of the arm 481. The rod 47 side end portion of each arm 481 is provided with the engagement portion 482.

The engagement portion 482 of one of the pair of arms 481 protrudes toward the Z2 side and is engaged with an engagement portion 501 (described later) of the holding member 50 from the Z1 side. The engagement portion 482 of the other of the pair of arms 481 protrudes toward the Z1 side and is engaged with the engagement portion 501 of the holding member 59 from the Z2 side.

Further, the shaft portion 481a of one of the pair of arms 481 protrudes toward the Z1 side. The shaft portion 481a of the one of the pair of arms 481 is rotatably supported by a frame member 52 (see FIG. 7) provided inside the housing 41. Specifically, the shaft portion 481a of the one of the pair of arms 481 is inserted in and is rotatably supported by a support portion 521 including a hole provided in the frame member 52. The shaft portion 481a of the other of the pair of arms 481 protrudes toward the Z2 side. The shaft portion 481a of the other of the pair of arms 481 is rotatably supported by the base 411. Specifically, the shaft portion 481a of the other of the pair of arms 481 is inserted in and rotatably supported by a support portion 411a including a recess provided in the base 411.

In an embodiment, the axis of the shaft portions 481a is disposed between the engagement portion 482 and the fourth drive shaft 44d in the X direction orthogonal to the Y direction. This allows the axis of the shaft portions 481a to be positioned between the engagement portion 482 and the fourth drive shaft 44d in the direction orthogonal to the Y direction, making the lever 48 more compact than in a case where the axis of the shaft portions 481a is positioned on the outer side of the fourth drive shaft 44d. The axis of the shaft portions 481a is disposed on the fourth drive shaft 44d side in the X direction from the center between the engagement portion 482 and the fourth drive shaft 44d.

In an embodiment, the driving force transmission part 49 is provided between the fourth driving shaft 44d and the lever 48, and transmits the driving force from the fourth driving shaft 44d to the lever 48. This allows the driving force to be transmitted from the fourth drive shaft 44d to the lever 48 via the driving force transmission part 49, so that the lever 48 can be disposed away from the fourth drive shaft 44d. As a result, even in cases where it is difficult to secure a space for providing the lever 48 near the fourth drive shaft 44d, the space for providing the lever 48 can be easily secured.

In an embodiment, the driving force transmission part 49 includes a gear train 491 including a spur gear 491a and a sector gear 491b, and transmits the driving force from the fourth drive shaft 44d to the lever 48 by the gear train 491. This allows the driving force to be reliably transmitted from the fourth drive shaft 44d to the lever 48 by the gear train 491, which transmits the driving force through meshing. Further, since the gear train 491 includes the sector gear 491b, the gear train 491 can be made more compact than in a case where a spur gear is provided instead of the sector gear 491b.

In an embodiment, the driving force transmission part 49 transmits the rotation of the fourth drive shaft 44d to the lever 48 with decelerating the speed of the rotation of the fourth drive shaft 44d. As a result, the lever 48 is driven with a torque that increases inversely proportional to the deceleration of the rotation of the fourth drive shaft 44d, so that the rod 47 can be easily moved in the Y direction by driving the lever 48. In addition, the driving of the lever 48 by the torque that increases inversely proportional to the deceleration of the rotation of the fourth drive shaft 44d is effective in the case where the spring member 51 is provided as in an embodiment.

The sector gear 491b meshes with a spur gear 44d1 provided to the fourth drive shaft 44d. The sector gear 491b has the number of teeth that reduces the rotation speed of the fourth drive shaft 44d. The spur gear 491a meshes with a sector gear 483a provided to the connection portion 483 of the lever 48. The spur gear 491a and the sector gear 491b are supported by a common rotation shaft 491c and rotate integrally with each other by the rotation shaft 491c.

In an embodiment, the holding member 50 holds the rod 47. The spring member 51 biases the holding member 50 toward the distal end side (Y1 side) in the Y direction. The engagement portions 482 are engaged with the rod 47 via the holding member 50, and when the engagement portions 482 push the holding member 50 toward the proximal end side (Y2 side) in the Y direction, a gripping force of the end effector 43 is generated by the reaction force of the spring member 51. Accordingly, it is possible to determine the gripping force of the end effector 43 by a spring constant of the spring member 51, so that the gripping force of the end effector 43 can be applied stably. Also, unlike a case where the gripping force of the end effector 43 is applied using a wire(s) as in the wrist joint 45, the gripping force is less susceptible to slight stretching of the wire. This eliminates the need to precisely set the tightening angles of the motors of the drivers 212.

The holding member 50 includes the engagement portion 501, a spring accommodation portion 502, and a rod holding portion 503. The engagement portion 501 of the holding member 50 is engaged with the engagement portions 482 of the lever 48. The engagement portion 501 is configured as a recess formed in a circumferential shape. A portion of the engagement portion 501 on the Z1 side is recessed toward the Z2 side, and is engaged with the engagement portion 482 of one of the pair of arms 481. A portion of the engagement portion 501 on the Z2 side is recessed toward the Z1 side, and is engaged with the engagement portion 482 of the other of the pair of arms 481. The spring accommodation portion 502 accommodates the spring member 51. The spring accommodation portion 502 includes a recess recessed toward the Y1 side. The spring accommodation portion 502 holds the spring member 51 between the spring accommodation portion 502 and a plate-shaped washer 53 provided on the Y2 side of the spring accommodation portion 502. The rod holding portion 503 holds a held portion 47a provided to the rod 47. The rod holding portion 503 includes a hole extending along the Y direction.

The spring member 51 is a compression coil spring having a predetermined spring constant. When the holding member 50 is moved in the Y2 direction by the lever 48, the spring member 51 is compressed between the spring accommodation portion 502 and the washer 53. When the holding member 50 is moved in the Y1 direction by the lever 48, the spring member 51 is stretched between the spring accommodation portion 502 and the washer 53.

Next, the operation of moving the rod 47 in the Y direction by the lever 48 is described. When the fourth drive shaft 44d is driven to rotate, the driving force from the fourth drive shaft 44d is transmitted to the lever 48 via the gear train 491 of the driving force transmission part 49. This causes the lever 48 to rotate about the axis of the shaft portion 481a of the lever 48. In the case where the rod 47 is moved in the Y1 direction, a Y1 side surface of the engagement portion 482 of the lever 48 abuts against a Y2 side surface of the engagement portion 501 of the holding member 50, and the holding member 50 is pushed and moved in the Y1 direction. With this, the rod 47, whose held portion 47a is held by the rod holding portion 503 of the holding member 50, is moved in the Y1 direction. This moves the first jaw 431 in the opening direction. To the contrary, in the case where the rod 47 is moved in the Y2 direction, a Y2 side surface of the engagement portion 482 of the lever 48 abuts against a Y1 side surface of the engagement portion 501 of the holding member 50, and the holding member 50 is pushed and moved in the Y2 direction. With this, the rod 47, whose held portion 47a is held by the rod holding portion 503 of the holding member 50, is moved in the Y2 direction. This moves the first jaw 431 in the closing direction.

### (Configuration of Mechanism for Fixing Memory Board)

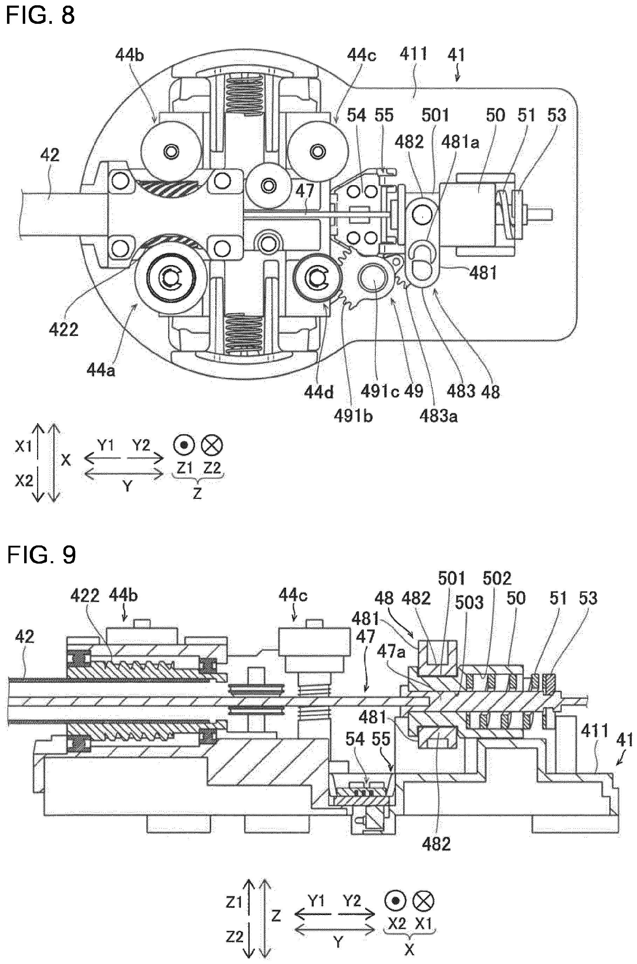

Next, a configuration of a mechanism 55 for fixing a circuit board 54 is described with reference to FIGS. 8, 15, and 16.

As illustrated in FIGS. 8, 15 and 16, the surgical instrument 40a includes the circuit board 54 and the fixing mechanism 55 for fixing the circuit board 54. The circuit board 54 is provided on the base 411. The circuit board 54 is a memory board. The circuit board 54 stores information about the surgical instrument 40a, such as a type of the surgical instrument 40a and the number of times the surgical instrument 40a has been used. The fixing mechanism 55 fixes the circuit board 54 to the base 411. The fixing mechanism 55 is a snap-fit fixing mechanism with a plurality (four pieces) of snap-fit portions 551. The snap fit portions 551 are provided two on each of the Y1 side and the Y2 side of the circuit board 54, and press an outer periphery of the circuit board 54 from the Z1 side. When the circuit board 54 is attached to the fixing mechanism 55 from the Z1 side toward the Z2 side, the snap fit portions 551 are pressed by the circuit board 54 and elastically deformed. By providing the snap-fit mechanism as the fixing mechanism 55, it is possible to fix the circuit board 54 to the base 411 with a simple structure.

### (Configuration of Wrist Joint)

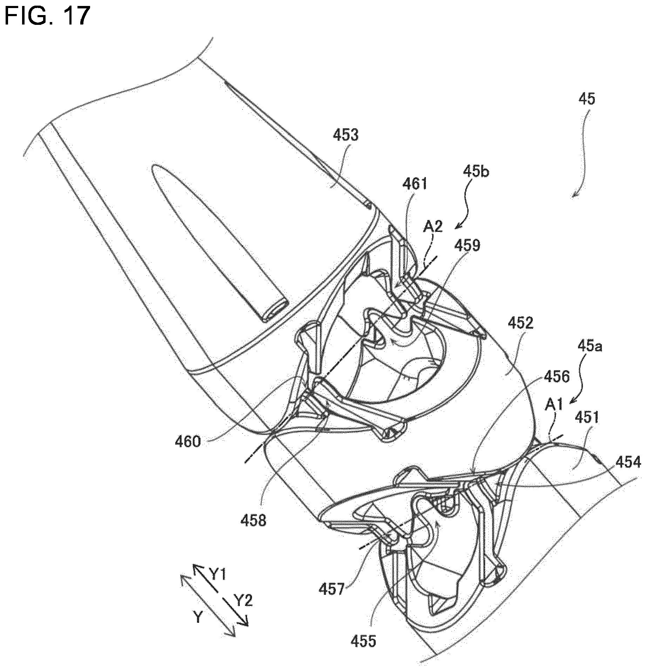

Next, a configuration of the wrist joint 45 is described with reference to FIGS. 17 to 26.

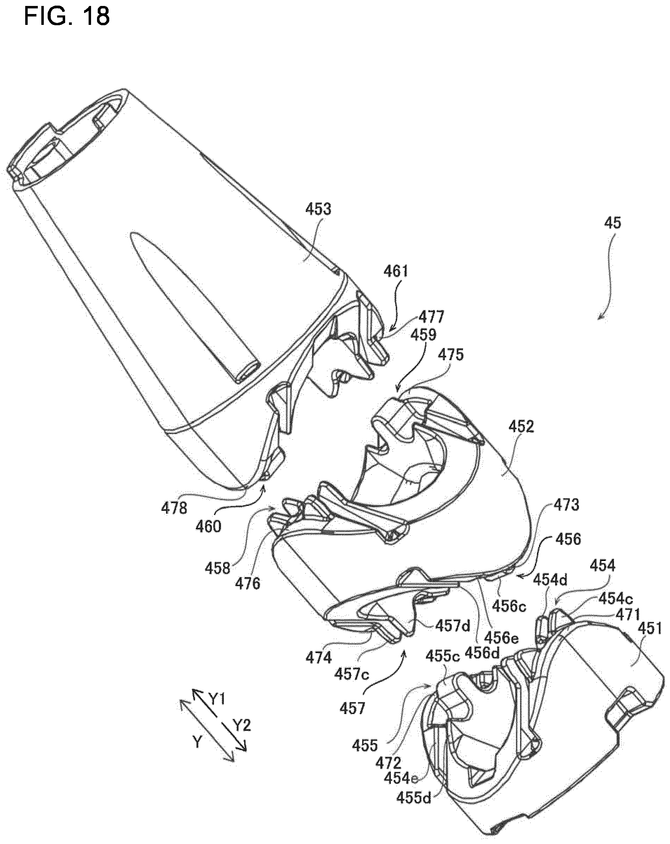

As illustrated in FIGS. 17 and 18, the wrist joint 45 includes the first joint component 451 and the second joint component 452 that is adjacent to the first joint component 451 on the distal side (Y1 side) and rotates relative to the first joint component 451. The wrist joint 45 also includes the third joint component 453 that is adjacent to the second joint component 452 on the distal side (Y1 side) and rotates relative to the second joint component 452. As illustrated in FIGS. 10 and 17, the second joint component 452 is rotatable relative to the first joint component 451 about the rotation axis A1. The rotation axis A1 extends in the direction orthogonal to the longitudinal direction (Y direction) of the shaft 42. Further, the third joint component 453 is rotatable relative to the second joint component 452 about the rotation axis A2 that is orthogonal to the rotation axis A1. The rotation axis A2 extends in the direction orthogonal to the rotation axis A1 when viewed in the longitudinal direction (Y direction) of the shaft 42.

The first joint component 451 includes a first meshing portion 454, a second meshing portion 455 arranged so as to be opposed to the first meshing portion 454 in a radial direction (A1 direction) orthogonal to the longitudinal direction of the shaft, a first contact portion 471 located radially outside of and adjacent to the first meshing portion 454, and a second contact portion 472 provided radially outside of and adjacent to the second meshing portion 455. The first meshing portion 454, the second meshing portion 455, the first contact portion 471, and the second contact portion 472 are provided at the end portion on the distal side (the Y1 side) of the first joint component 451.

The second joint component 452 includes a third meshing portion 456 that meshes with the first meshing portion 454, a fourth meshing portion 457 that is provided so as to be opposed to the third meshing portion 456 in the radial direction (A1 direction) and meshes with the second meshing portion 455, a third contact portion 473 that is in contact with the first contact portion 471, and a fourth contact portion 474 that is in contact with the second contact portion 472. The third meshing portion 456, the fourth meshing portion 457, the third contact portion 473 and the fourth contact portion 474 are provided at the end portion on the proximal side (Y2 side) of the second joint component 452. The contact between the first contact portion 471 and the third contact portion 473 reduces the load between the first meshing portion 454 and the third meshing portion 456. The contact between the second contact portion 472 and the fourth contact portion 474 reduces the load between the second meshing portion 455 and the fourth meshing portion 457.

The second joint component 452 also includes a fifth meshing portion 458, a sixth meshing portion 459 provided so as to be opposed to the fifth meshing portion 458 in the radial direction (A2 direction), a fifth contact portion 475 located radially outward of and adjacent to the fifth meshing portion 458, and a sixth contact portion 476 provided radially outside of and adjacent to the sixth meshing portion 459. The fifth meshing portion 458, the sixth meshing portion 459, the fifth contact portion 475 and the sixth contact portion 476 are provided at the end portion on the distal side (the Y1 side) of the second joint component 452.

The third joint component 453 includes a seventh meshing portion 460 that meshes with the fifth meshing portion 458, an eighth meshing portion 461 that is arranged so as to be opposed to the seventh meshing portion 460 in the radial direction (A2 direction) and meshes with the sixth meshing portion 459, a seventh contact portion 477 that is in contact with the fifth contact portion 475, and an eighth contact portion 478 that is in contact with the sixth contact portion 476. The seventh meshing portion 460, the eighth meshing portion 461, the seventh contact portion 477, and the eighth contact portion 478 are provided at the end portion on the proximal side (the Y2 side) of the third joint component 453. The contact between the fifth contact portion 475 and the seventh contact portion 477 reduces the load between the fifth meshing portion 458 and the seventh meshing portion 460. The contact between the sixth contact portion 476 and the eighth contact portion 478 reduces the load between the sixth meshing portion 459 and the eighth meshing portion 461.

As illustrated in FIG. 18, the first meshing portion 454 and the second meshing portion 455 have different shapes from each other. The third meshing portion 456 and the fourth meshing portion 457 have different shapes from each other. This makes it difficult for the first meshing portion 454 of the first joint component 451 and the fourth meshing portion 457 of the second joint component 452 to mesh with each other, and makes it difficult for the second meshing portion 455 of the first joint component 451 and the third meshing portion 456 of the second joint component 452 to mesh with each other. This allows the first and third meshing portions 454 and 456 to be properly meshed, and the second and fourth meshing portions 455 and 457 to be properly meshed, when the first and second joint components 451 and 452 are assembled together. As a result, the wrist joint can be easily and accurately assembled, and even if a problem occurs, the cause of the problem can be easily identified, allowing for appropriate quality control of the surgical instrument.

Further, the fifth meshing portion 458 and the sixth meshing portion 459 have shapes different from each other. The seventh meshing portion 460 and the eighth meshing portion 461 have shapes different from each other. This also enables accurate assembly of the wrist joint and appropriate quality control of the surgical instrument.

Further, the first meshing portion 454 and the fourth meshing portion 457 have the same shape as each other. The second meshing portion 455 and the third meshing portion 456 have the same shape as each other. This allows the meshing structure of the first meshing portion 454 and the third meshing portion 456 to be the same as the meshing structure of the second meshing portion 455 and the fourth meshing portion 457, so that the joint movement of the wrist joint 45 can be supported in a well-balanced manner by the two meshing structures between the first joint component 451 and the second joint component 452.

The fifth meshing portion 458 and the eighth meshing portion 461 have the same shape as each other. The sixth meshing portion 459 and the seventh meshing portion 460 have the same shape as each other. This also makes it possible to support the joint movement of the wrist joint 45 in a well-balanced manner with the two meshing structures between the second joint component 452 and the third joint component 453.

Moreover, the first meshing portion 454, the fourth meshing portion 457, the fifth meshing portion 458, and the eighth meshing portion 461 have the same shape as one another. The second meshing portion 455, the third meshing portion 456, the sixth meshing portion 459 and the seventh meshing portion 460 have the same shape as one another. In other words, the first meshing portion 454 and the fifth meshing portion 458 have the same configuration as each other, and the second meshing portion 455 and the sixth meshing portion 459 have the same configuration as each other. Further, the third meshing portion 456 and the seventh meshing portion 460 have the same configuration as each other, and the fourth meshing portion 457 and the eighth meshing portion 461 have the same configuration as each other. Accordingly, the description of the fifth meshing portion 458, the sixth meshing portion 459, the seventh meshing portion 460, and the eighth meshing portion 461 will be omitted as appropriate for eliminating redundancy.

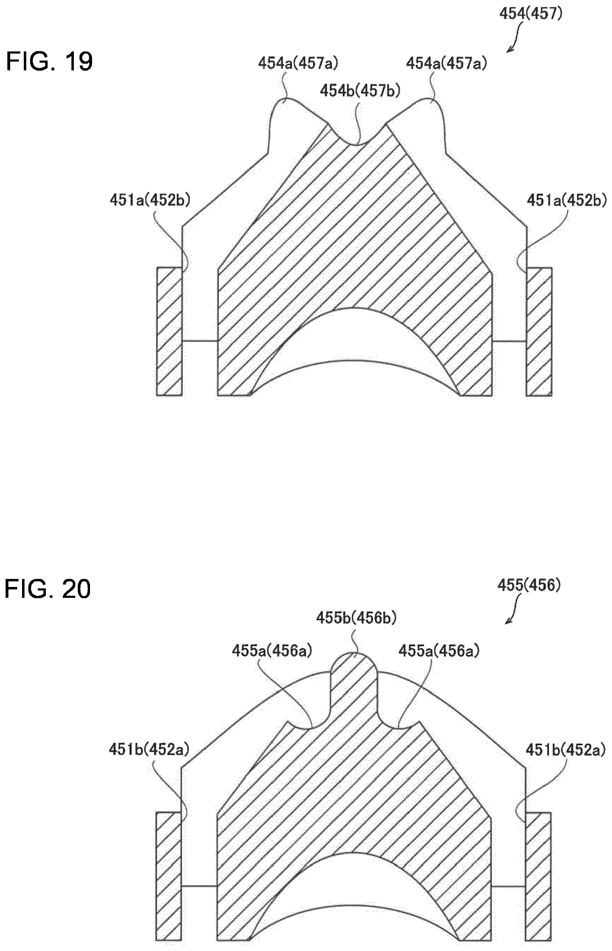

As illustrated in FIG. 19, the first meshing portion 454 includes a pair of first protrusions 454a arranged along the rotation direction of the second joint component 452, and a first recess 454b arranged between the pair of first protrusions 454a. In the same manner, the fourth meshing portion 457 includes a pair of fourth protrusions 457a arranged along the rotation direction of the second joint component 452, and a fourth recess 457b arranged between the pair of fourth protrusions 457a.

As illustrated in FIG. 20, the second meshing portion 455 includes a pair of second recesses 455a arranged along the rotation direction of the second joint component 452, and a second protrusion 455b arranged between the pair of second recesses 455a. In the same manner, the third meshing portion 456 includes a pair of third recesses 456a arranged along the rotation direction of the second joint component 452, and a third protrusion 456b arranged between the pair of third recesses 456a.

This makes it possible to prevent the first meshing portion 454 having the first recess 454b and the fourth meshing portion 457 having the fourth recess 457b from easily meshing with each other, and also makes it possible to prevent the second meshing portion 455 having the second protrusion 455b and the third meshing portion 456 having the third protrusion 456b from easily meshing with each other. As a result, it is possible to effectively prevent the first joint component 451 and the second joint component 452 from being assembled in a wrong direction.

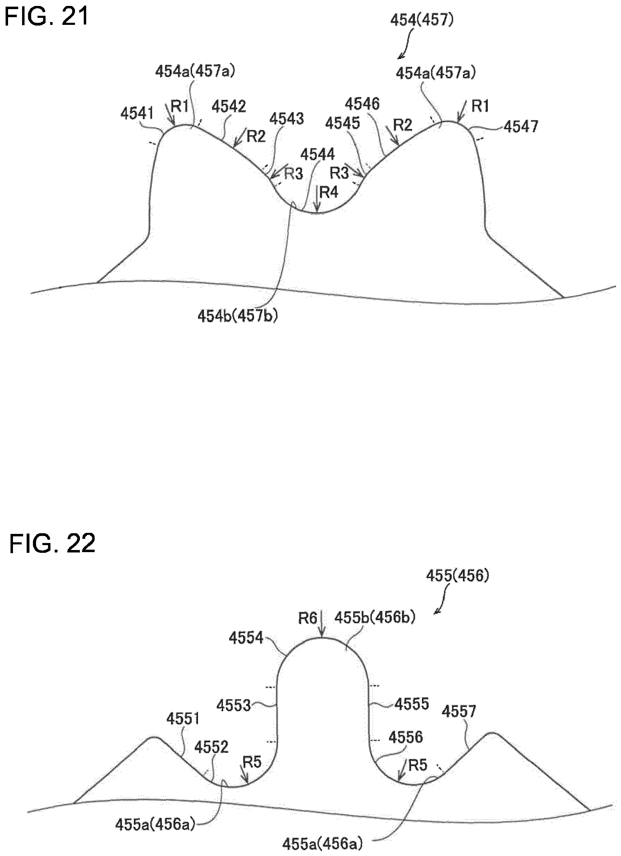

As illustrated in FIGS. 21 and 22, each of the first meshing portion 454, the second meshing portion 455, the third meshing portion 456 and the fourth meshing portion 457 have a shape in which arcs are connected together, or a shape in which arcs and straight lines are connected together, when viewed in the rotation axis direction of the second joint component 452 (A1 direction). This makes it possible to easily design the first meshing portion 454, the second meshing portion 455, the third meshing portion 456, and the fourth meshing portion 457. In addition, the shapes of the first meshing portion 454, the second meshing portion 455, the third meshing portion 456, and the fourth meshing portion 457 can be easily inspected, so that the shapes of the first meshing portion 454, the second meshing portion 455, the third meshing portion 456, and the fourth meshing portion 457 can be more appropriately managed.

Specifically, the first meshing portion 454 and the fourth meshing portion 457 each have a shape in which arcs are connected, as illustrated in FIG. 21. The first meshing portion 454 and the fourth meshing portion 457 each have an outer shape formed by connecting lines including an arc 4541 having a radius R1, an arc 4542 having a radius R2 connected to the arc 4541, an arc 4543 having a radius R3 connected to the arc 4542, an arc 4544 having a radius R4 connected to the arc 4543, an arc 4545 having the radius R3 connected to the arc 4544, an arc 4546 having the radius R2 connected to the arc 4545, and an arc 4547 having the radius R1 connected to the arc 4546. Further, the first meshing portion 454 and the fourth meshing portion 457 each have a linear symmetric shape when viewed along the rotation axis direction of the second joint component 452 (A1 direction).

As illustrated in FIG. 22, the second and third meshing portions 455 and 456 each have a shape in which arcs and straight lines are connected. The second meshing portion 455 and the third meshing portion 456 each have an outer shape formed by connecting lines including a line segment 4551, an arc 4552 having a radius R5 connected to line segment 4551, a line segment 4553 connected to the arc 4552, an arc 4554 having a radius R6 connected to the line segment 4553, a line segment 4555 connected to the arc 4554, an arc 4556 having the radius R5 connected to the line segment 4555, and a line segment 4557 connected to the arc 4556. Further, the second meshing portion 455 and the third meshing portion 456 each have a linear symmetric shape when viewed in the rotation axis direction (A1 direction) of the second joint component 452.

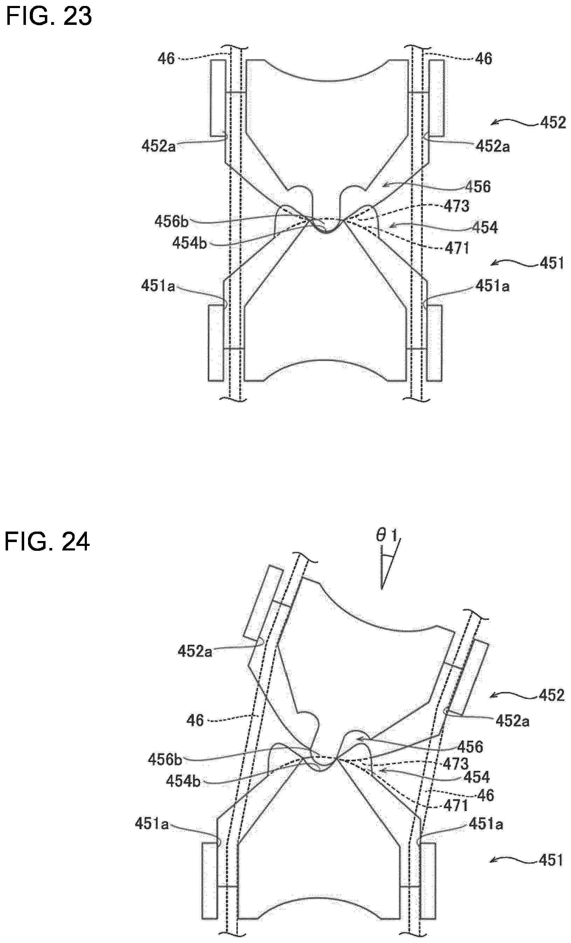

As illustrated in FIGS. 23 to 26, the wrist joint 45 is articulated by driving the wires 46 to rotate the second joint component 452 relative to the first joint component 451. As illustrated in FIG. 10, the wire 46 has the end portion 46e thereof fixed to the third joint component 453 on the end effector side (Y1 side) of the wrist joint 45.

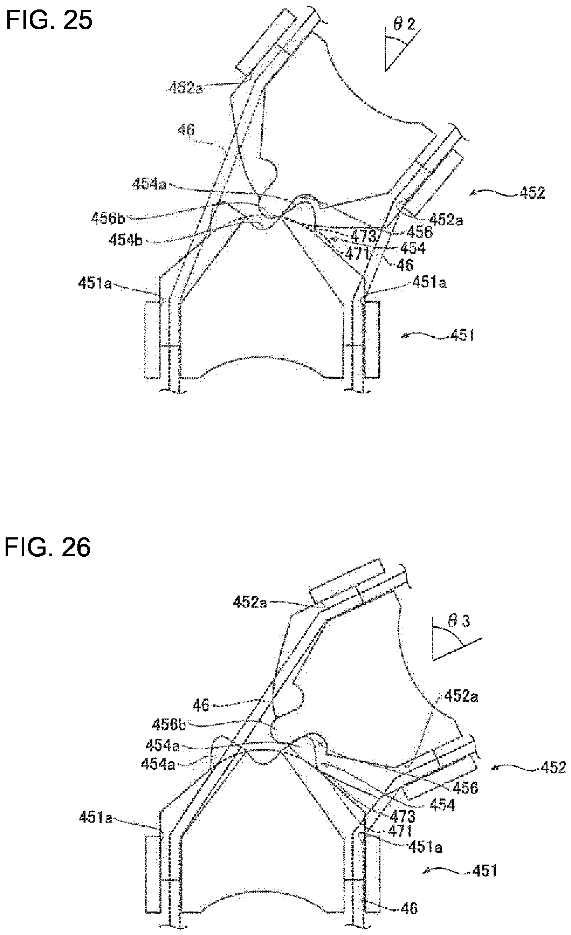

As illustrated in FIG. 18, the first meshing portion 454 includes a first portion 454c and a second portion 454d that are separated in the radial direction (A1 direction) by a distance that allows the wires 46 to pass therethrough. Further, each of the first portion 454c and the second portion 454d is formed with the first protrusions 454a and the first recess 454b. In the same manner as the first meshing portion 454, the fourth meshing portion 457 includes a third portion 457c and a fourth portion 457d that are separated in the radial direction (A1 direction) by a distance that allows the wires 46 to pass through. Further, each of the third portion 457c and the fourth portion 457d is formed with the fourth protrusions 457a and the fourth recess 457b. This prevents the wires 46 from interfering with the first portion 454c and the second portion 454d when the joint movement performed in which the second joint component 452 is rotated significantly relative to the first joint component 451 such as being illustrated in FIG. 26.

As illustrated in FIG. 18, the second meshing portion 455 includes a fifth portion 455c that is continuously formed in the radial direction (A1 direction). The second meshing portion 455 also includes a portion 455d and a portion 455e that are separated in the radial direction (A1 direction) by a distance that allows the wires 46 to pass therethrough. Further, the second protrusion 455b is formed in the fifth portion 455c. Further, the second recesses 455a are formed in each of the portion 455d and the portion 454e.

As in the second meshing portion 455, the third meshing portion 456 includes a sixth portion 456c that is formed continuously in the radial direction (A1 direction). The third meshing portion 456 also includes a portion 456d and a portion 456e that are separated in the radial direction (A1 direction) by a distance that allows the wires 46 to pass therethrough. Further, the third protrusion 456b is formed in the sixth portion 456c. Further, the third recesses 456a are formed in each of the portion 456d and the portion 456e.

As illustrated in FIG. 19, the first joint component 451 includes first through holes 451a which are disposed at positions spaced apart from the first meshing portion 454 and through which the wires 46 pass. As illustrated in FIG. 20, the first joint component 451 includes second through holes 451b which are disposed at positions spaced apart from the second meshing portion 455 and through which the wires 46 passes. As illustrated in FIG. 20, the second joint component 452 includes third through holes 452a which are disposed at positions spaced apart from the third meshing portion 456 and through which the wires 46 pass. As illustrated in FIG. 19, the second joint component 452 includes fourth through holes 452b which are disposed at positions spaced apart from the fourth meshing portion 457 and through which the wires 46 pass. This allows the through holes through which the wires 46 pass to be located at the positions away from the meshing portions, thereby preventing the meshing portions from interfering with the wires 46.

Next, with reference to FIGS. 23 to 26, the rotation of the second joint component 452 relative to the first joint component 451 is described. Although FIGS. 23 to 26 illustrate the relationship between the first meshing portion 454 of the first joint component 451 and the third meshing portion 456 of the second joint component 452, the relationship between the second meshing portion 455 of the first joint component 451 and the fourth meshing portion 457 of the second joint component 452 is in a same manner with the protrusions and the recesses interchanged, and thus illustration and description thereof is omitted for eliminating redundancy. Also the rotation of the third joint component 453 relative to the second joint component 452 takes the same or similar operation, and thus illustration and description thereof is omitted for eliminate redundancy.

As illustrated in FIG. 23, when the second joint component 452 is not rotated relative to the first joint component 451, the third protrusion 456b of the third meshing portion 456 of the second joint component 452 is fit in the first recess 454b of the first meshing portion 454 of the first joint component 451.

As illustrated in FIG. 24, by the operation of the wires 46, the third protrusion 456b of the third meshing portion 456 of the second joint component 452 is rotated with respect to the first recess 454b of the first meshing portion 454 of the first joint component 451 while contacting with the first recess 454b, so that the second joint component 452 is rotated by an angle θ1 relative to the first joint component 451.

As illustrated in FIG. 25, by the operation of the wires 46, the third protrusion 456b of the third meshing portion 456 of the second joint component 452 is rotated with respect to the first protrusion 454a of the first meshing portion 454 of the first joint component 451 while contacting with the first protrusion 454a, so that the second joint component 452 is rotated by an angle θ2 relative to the first joint component 451. The angle θ2 is greater than the angle θ1.

As illustrated in FIG. 26, by the operation of the wires 46, the third protrusion 456b of the third meshing portion 456 of the second joint component 452 is further rotated with respect to the first protrusion 454a of the first meshing portion 454 of the first joint component 451 while contacting with the first protrusion 454a, so that the second joint component 452 is rotated by an angle θ3 relative to the first joint component 451. The angle θ3 is greater than the angle θ2.

### (Modifications)

Note that one or more embodiments disclosed herein should be considered as exemplary in all respects and do not limit the invention. The scope of the invention is indicated by claims, not by explanation of one or more embodiments described above, and includes equivalents to the claims and all alterations (modification) within the same.

For example, in one or more embodiments described above, a case has been described in which the four drive shafts are provided, but the invention is not limited thereto. For example, five or more drive shafts may be provided.

Further, in one or more embodiments described above, a case has been described in which the rod is provided as the elongate element, but the invention is not limited thereto. For example, the elongate element may be a wire or a cable.

Further, in one or more embodiments described above, a case has been described in which the driving force transmission part is provided between the fourth drive shaft and the lever, but the invention is not limited thereto. For example, the lever may be directly connected to the fourth drive shaft.

Further, in one or more embodiments described above, a case has been described in which the driving force transmission part includes the gear train, but the invention is not limited thereto. For example, the driving force transmission part may include a belt pulley or the like other than the gear train.

Further, in one or more embodiments described above, a case has been described in which the gear train includes the spur gear and the sector gear, but the invention is not limited thereto. For example, the gear train may include only a spur gear or only a sector gear. Also, the gear train may include a gear(s) other than the spur gear and the sector gear.

Further, in one or more embodiments described above, a case has been described in which the spring member is provided, but the invention is not limited thereto. For example, the spring member may not be provided.

### (Aspects)

It will be understood by those skilled in the art that the exemplary embodiments described above are embodiments of the following aspects.
(Item 1) A surgical instrument comprising:
   a housing including a base that is to be attached to a robot arm;
   a shaft including a proximal end connected to the base;
   a wrist joint connected to a distal end side of the shaft, the wrist joint including a first joint configured to perform a pitch joint movement about a first rotation axis intersecting a longitudinal direction of the shaft and a second joint configured to perform a yaw joint movement about a second rotation axis intersecting the longitudinal direction and the first rotation axis;
   an end effector connected to a distal end of the wrist joint; and
      at least four drive shafts provided to the base and configured to be driven to rotate by driving forces from motors provided to the robot arm, wherein
   the at least four drive shafts comprise a first drive shaft configured to roll the shaft about an axis along the longitudinal direction of the shaft, a second drive shaft and a third drive shaft configured to pitch the first joint and yaw the second joint, and a fourth drive shaft configured to drive the end effector,
   the wrist joint includes a first joint component and a second joint component that is adjacent to the first joint component and provided on a distal side of the first joint component and is rotatable relative to the first joint component,
   the first joint component includes a first meshing portion and a second meshing portion provided to be opposed to the first meshing portion in a radial direction orthogonal to the longitudinal direction of the shaft,
   the second joint component includes a third meshing portion that meshes with the first meshing portion, and a fourth meshing portion that is provided to be opposed to the third meshing portion in the radial direction and meshes with the second meshing portion,
   the first meshing portion and the second meshing portion have different shapes from each other, and
   the third meshing portion and the fourth meshing portion have different shapes from each other.
(Item 2) The surgical instrument according to item 1, wherein
   the first meshing portion and the fourth meshing portion have a same shape as each other, and
   the second meshing portion and the third meshing portion have a same shape as each other.
(Item 3) The surgical instrument according to item 1 or 2, wherein
   the first meshing portion includes a pair of first protrusions arranged along a rotation direction of the second joint component, and a first recess arranged between the pair of first protrusions,
   the second meshing portion includes a pair of second recesses arranged along the rotation direction of the second joint component and a second protrusion arranged between the pair of second recesses,
   the third meshing portion includes a pair of third recesses arranged along the rotation direction of the second joint component and a third protrusion arranged between the pair of third recesses, and
   the fourth meshing portion includes a pair of fourth protrusions arranged along the rotation direction of the second joint component, and a fourth recess arranged between the pair of fourth protrusions.
(Item 4) The surgical instrument according to any one of items 1 to 3, wherein
   the first meshing portion, the second meshing portion, the third meshing portion, and the fourth meshing portion each have a shape in which arcs are connected together, or a shape in which arcs and straight lines are connected together, in a view along a direction of a rotation axis of the second joint component.
(Item 5) The surgical instrument according to any one of items 1 to 4, further comprising a wire configured to rotate the second joint component with respect to the first joint component, wherein
   the first meshing portion includes a first portion and a second portion which are divided and arranged so as to sandwich the wire in the radial direction;
   the fourth meshing portion includes a third portion and a fourth portion which are divided and arranged so as to sandwich the wire in the radial direction.
(Item 6) The surgical instrument according to item 5, wherein
   the second meshing portion includes a fifth portion formed continuously in the radial direction, and
   the third meshing portion includes a sixth portion formed continuously in the radial direction.
(Item 7) The surgical instrument according to item 5 or 6, wherein
   the first joint component includes a first through hole arranged at a position separated from the first meshing portion and through which the wire is passed, and a second through hole arranged at a position separated from the second meshing portion and through which the wire is passed,
   the second joint component includes a third through hole arranged at a position separated from the third meshing portion and through which the wire is passed, and a fourth through hole arranged at a position separated from the fourth meshing portion and through which the wire is passed.
(Item 8) The surgical instrument according to any one of items 5 to 7, wherein
   the wrist joint includes a third joint component connected to the second joint component through the second joint, and
   the wire includes a distal end fixed to the third joint component.
(Item 9) The surgical instrument according to any one of items 1 to 7, wherein
   the wrist joint includes a third joint component connected to the second joint component through the second joint,
   the second joint component includes a fifth meshing portion and a sixth meshing portion provided to be opposed to the fifth meshing portion in the radial direction,
   the third joint component includes a seventh meshing portion meshing with the fifth meshing portion, and an eighth meshing portion provided to be opposed to the seventh meshing portion in the radial direction and meshing with the sixth meshing portion,
   the fifth meshing portion and the sixth meshing portion have different shapes from each other, and
   the seventh meshing portion and the eighth meshing portion have different shapes from each other.

## Claims

1. A surgical instrument (40a) comprising:
a housing (41) including a base (411) that is to be attached to a robot arm (21a),
a shaft (42) including a proximal end connected to the base;
a wrist joint (45) connected to a distal end side of the shaft, the wrist joint including a first joint (45a) configured to perform a pitch joint movement about a first rotation axis (A1) intersecting a longitudinal direction of the shaft and a second joint (45b) configured to perform a yaw joint movement about a second rotation axis (A2) intersecting the longitudinal direction and the first rotation axis;
an end effector (43) connected to a distal end of the wrist joint;
an elongate element (47) extending in the longitudinal direction of the shaft, the elongate element including a distal end thereof connected to the end effector;
at least four drive shafts (44a, 44b, 44c, 44d) provided to the base and configured to driven to rotate by driving forces from motors provided to the robot arm; and
a lever (48) configured to move the elongate element in the longitudinal direction, wherein
the at least four drive shafts comprise a first drive shaft (44a) configured to roll the shaft about an axis along the longitudinal direction of the shaft, a second drive shaft (44b) and a third drive shaft (44c) configured to pitch the first joint and yaw the second joint, and a fourth drive shaft (44d) configured to move the elongate element in the longitudinal direction of the shaft, and
the lever includes an arm (481), an engagement portion (482) that is provided to one side of the arm and is engaged with the elongate element, and a shaft portion (481a) that is provided to the other side of the arm and is rotatably arranged on the base so as to move the engagement portion by the driving force from the fourth drive shaft.

2. The surgical instrument according to claim 1, further comprising
a driving force transmission part (49) provided between the fourth drive shaft and the lever and configured to transmit the driving force from the fourth drive shaft to the lever.

3. The surgical instrument according to claim 2, wherein
the driving force transmission part includes a gear train (491) including a spur gear (491a) and a sector gear (491b), and is configured to transmit the driving force from the fourth drive shaft to the lever through the gear train.

4. The surgical instrument according to claim 2 or 3, wherein
the driving force transmission part is configured to transmit rotation of the fourth driving shaft to the lever with decelerating a speed of the rotation of the fourth drive shaft.

5. The surgical instrument according to any one of claims 1 to 4, wherein
the arm comprises a first arm (481), a second arm (481) opposed to the first arm and a connection portion (483) connecting one side of the first arm and one side of the second arm,
the engagement portion comprises a first engagement portion (482) provided to the other side of the first arm and a second engagement portion (482) provided to the other side of the second arm, and
the elongate element is arranged between the first engagement portion and the second engagement portion such that the elongate element engages with the first engagement portion and the second engagement portion.

6. The surgical instrument according to claim 5, further comprising
a holding member (50) that holds the elongate element, wherein
the holding member (50) is arranged between the first engagement portion and the second engagement portion such that the holding member engages with the first engagement portion and the second engagement portion.

7. The surgical instrument according to any one of claims 1 to 6, wherein
the end effector includes a first jaw (431) and a second jaw (432), and
the first jaw includes a support shaft (431a) rotatably supported by the second jaw and a second connection portion (431b) connected to a distal end of the elongate element, and is configured to rotate about an axis of the support shaft due to the movement of the elongate element in the longitudinal direction.

8. The surgical instrument according to any one of claims 1 to 7, further comprising
a holding member (50) that holds the elongate element; and
a spring member (51) that biases the holding member toward the distal end side of the shaft in the longitudinal direction, wherein
the engagement portion is engaged with the elongate element via the holding member.

9. The surgical instrument according to any one of claims 1 to 8, wherein
the elongate element is a rod.

10. The surgical instrument according to any one of claims 1 to 9, further comprising
first to fourth wires (46a, 46b, 46c, 46d) extending in the longitudinal direction of the shaft, wherein
the wrist joint includes a first joint component (451) connected to the distal end side of the shaft, a second joint component (452) connected to the first joint component via the first joint, and a third joint component (453) connected to the second joint component via the second joint, and
the first to fourth wires includes distal ends thereof fixed to the third joint component.

11. The surgical instrument according to claim 10, wherein
a cross section of the third joint component includes four regions (D1, D2, D3, D4) defined by a first plane (B1) parallel to both the longitudinal direction of the shaft and the first rotation axis and a second plane (B2) parallel to both the longitudinal direction of the shaft and the second rotation axis,
the first to fourth wires pass through the four regions, respectively, and
proximal ends of the first wire and the second wire are wound around and fixed to the second drive shaft, and proximal ends of the third wire and the fourth wire are wound around and fixed to the third drive shaft.

12. The surgical instrument according to claim 10 or 11, wherein
the second drive shaft is configured to rotate in a first direction to apply tension to the first wire and to rotate in a second direction opposite the first direction to apply tension to the second wire,
the third drive shaft is configured to rotate in a third direction to apply tension to the third wire and to rotate in a fourth direction opposite to the third direction to apply tension to the fourth wire,
the first joint is configured, when the rotation of the second drive shaft applies the tension to the first wire and the rotation of the third drive shaft applies the tension to the fourth wire, or when the rotation of the second drive shaft applies the tension to the second wire and the rotation of the third drive shaft applies the tension to the third wire, to perform the pitch joint movement about the first rotation axis, and
the second joint is configured, when the rotation of the second drive shaft applies the tension to the first wire and the rotation of the third drive shaft applies the tension to the third wire, or when the rotation of the second drive shaft applies the tension to the second wire and the rotation of the third drive shaft applies the tension to the fourth wire, to perform the yaw joint movement around the second rotation axis.

13. The surgical instrument according to any one of claims 1 to 12, wherein
the wrist joint includes a first joint component (451) connected to the distal end side of the shaft and a second joint component (452) connected to the first joint component via the first joint, and
the first joint component includes a first meshing portion (454) and a second meshing portion (455) provided to be opposed to the first meshing portion in a radial direction orthogonal to the longitudinal direction of the shaft,
the second joint component includes a third meshing portion (456) that meshes with the first meshing portion, and a fourth meshing portion (457) that is provided to be opposed to the third meshing portion in the radial direction and meshes with the second meshing portion,
the first and second meshing portions have different shapes from each other,
the third meshing portion and the fourth meshing portion have different shapes from each other,
the first meshing portion and the fourth meshing portion have a same shape as each other, and
the second meshing portion and the third meshing portion have a same shape as each other.

14. The surgical instrument according to claim 13, wherein
the first meshing portion includes a pair of first protrusions (454a) arranged along a rotation direction of the second joint component, and a first recess (454b) arranged between the pair of first protrusions,
the second meshing portion includes a pair of second recesses (455a) arranged along the rotation direction of the second joint component and a second protrusion (455b) arranged between the pair of second recesses,
the third meshing portion includes a pair of third recesses (456a) arranged along the rotation direction of the second joint component and a third protrusion (456b) arranged between the pair of third recesses, and
the fourth meshing portion includes a pair of fourth protrusions (457a) arranged along the rotation direction of the second joint component, and a fourth recess (457b) arranged between the pair of fourth protrusions.

15. The surgical instrument according to claim 13 or 14, wherein
the wrist joint includes a third joint component (453) connected to the second joint component via the second joint,
the second joint component includes a fifth meshing portion (458) and a sixth meshing portion (459) provided to be opposed to the fifth meshing portion in the radial direction,
the third joint component includes a seventh meshing portion (460) meshing with the fifth meshing portion (461), and an eighth meshing portion provided to be opposed to the seventh meshing portion in the radial direction and meshing with the sixth meshing portion,
the fifth meshing portion and the sixth meshing portion have different shapes from each other,
the seventh meshing portion and the eighth meshing portion have different shapes from each other,
the fifth meshing portion and the eighth meshing portion have a same shape as each other, and
the sixth meshing portion and the seventh meshing portion have a same shape as each other.
